# EUROPEAN PATENT APPLICATION

(11) **EP 1 604 664 A1**
(43) Date of publication of application: **14.12.2005**
(21) Application number: 04706359.9
(22) Date of filing: 29.01.2004
(51) Int. Cl.: A61K 31/4422, A61K 45/06, A61P 9/00, A61P 9/10, A61P 43/00, A61P 9/12, A61P 13/12

(54) **MEDICINE FOR PREVENTION OF AND TREATMENT FOR ARTERIOSCLEROSIS AND HYPERTENSION**

(30) Priority: 31.01.2003 JP 2003022990; 07.02.2003 JP 2003030830
(71) Applicant: Sankyo Company, Limited, Tokyo 103-8426 (JP)
(72) Inventor: HORIUCHI, Masatsugu, Onsen-gun, Ehime 791-0204 (JP); IWAI, Masaru, Shigenobu-cho, Onsen-gun, Ehime 791-0204 (JP); SADA, Toshio, Sankyo Company, Limited, Tokyo 140-8710 (JP); MIZUNO, Makoto, Sankyo Company, Limited, Tokyo 140-8710 (JP)
(74) Representative: Gibson, Christian John Robert
(86) International application number: PCT/JP2004/000861
(87) International publication number: WO 2004/067003

(57) **Abstract**

A medicament comprising the following composition: (A) an angiotensin II receptor antagonist selected from the group of a compound having a general formula (I), pharmacologically acceptable esters thereof and pharmacologically acceptable salts thereof (for example, olmesartan medoxomil and the like); 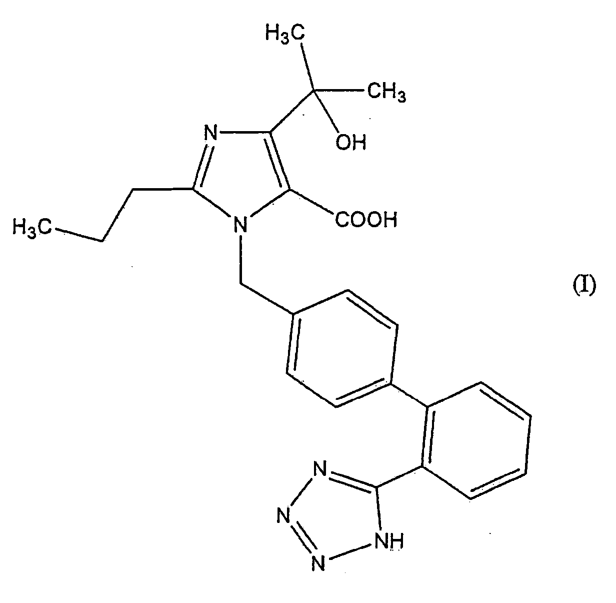 and (B) a calcium channel blocker selected from the group consisting of 1,4-dihydropyridine derivatives and pharmacologically acceptable salts thereof as active ingredients (for example, azelnidipine and the like) for the prophylaxis and/or treatment of arteriosclerosis, hypertension, heart diseases, renal diseases or cerebrovascular diseases.

## Description

### [Technical field]

The present invention relates to a medicament for the prophylaxis and/or treatment of arteriosclerosis. In addition, the present invention relates to a medicament for the prophylaxis and/or medical treatment of diseases such as hypertension, heart diseases (angina pectoris, myocardial infarction, arrhythmia (including sudden death), cardiac failure or cardiac hypertrophy), renal diseases (diabetic nephropathy, glomerulonephritis or nephrosclerosis) or cerebrovascular diseases (cerebral infarction or cerebral hemorrhage).

### [Background art]

Currently, calcium channel blockers and inhibitors of the renin-angiotensin system are widely used clinically for the prophylaxis and treatment of hypertension. Various types of calcium channel blockers are used, and among them 1,4-dihydropyridine derivatives such as amlodipine, benidipine, nitrendipine, manidipine, nicardipine, nifedipine, nisoldipine, cilnidipine, lercanidipine, niguldipine, nimodipine, aranidipine, efonidipine, barnidipine, felodipine, nilvadipine, azelnidipine and the like are long-lasting calcium channel blockers and are widely used clinically as first-choice antihypertensive agents. Furthermore, as inhibitors of the renin-angiotensin system, clinical use of angiotensin II receptor antagonists is growing larger and larger since, first, angiotensin II receptor antagonists lack side effects such as cough, which has been a cause of troubles elicited by angiotensin converting enzyme (ACE) inhibitors, and second, they exert protective effects on the cardiovascular and renal systems. However, the blood pressure of patients with hypertension cannot be fully controlled by only one kind of these drugs in many cases.

Since calcium channel blockers exert natriuretic action in addition to vasodilative action, they are also effective against hypertension caused by retention of fluid (renin-independent hypertension). On the other hand, angiotensin II receptor antagonists are particularly effective against renin-dependent hypertension, and in addition, they exert excellent protective activities in several organs. Therefore stable and significant antihypertensive effects are expected by combined administration of a calcium channel blocker and an angiotensin II receptor antagonist, no matter what the cause of hypertension.

Many combination drugs comprising a calcium channel blocker and an angiotensin II receptor antagonist have been proposed (for example, International Publication Number 01/15674 Official Gazette, International Publication Number 01/78699 Official Gazette, International Publication Number 02/43807 Official Gazette, International Publication Number 01/76632 Official Gazette, International Publication Number 01/74390 Official Gazette, Japanese Patent Publication (Kohyo) Number 2002-524408, International Publication Number 92/10097 Official Gazette, Japanese Patent Publication (Kokoku) Number Hei 7-035372, United Kingdom Patent Application Publication Number 2268743 Specification, Japanese Patent Publication (Kokai) Number Hei 6-56789, Japanese Patent Publication (Kokai) Number Hei 5-155867, United States Patent Application Publication Number 2001/0004640 Specification, USP 6204281 Specification, Japanese Patent Number 3057471, Japanese Patent Number 2930252, Japanese Patent Publication (Kohyo) Number 2002-507213, Japanese Patent Publication (Kohyo) Number 2001-513498, Japanese Patent Publication (Kohyo) Number 2000-508632, Japanese Patent Publication (Kokoku) Number Hei 7-91299, Japanese Patent Publication (Kokoku) Number Hei 7-14939, Japanese Patent Publication (Kokai) Number Hei 6-65207, Japanese Patent Publication (Kokai) Number Hei 5-213894, Japanese Patent Publication (Kohyo) Number 2002-518417, Japanese Patent Publication (Kohyo) Number 2002-506010, and Japanese Patent Publication (Kohyo) Number 2001-522872), and it is disclosed that optimum antihypertensive effects are achieved by combined administration of a specific calcium channel blocker and a specific angiotensin II receptor antagonist in some of these publications described above. However, the effects of combined administration of a specific angiotensin II receptor antagonist and a specific calcium channel blocker of the present invention are not known.

On the other hand, characteristics of pathological changes at the early stages of arteriosclerosis are abnormal thickening of the middle arteries or large arteries, and the pathological changes at the early stage of arteriosclerosis are characterized by injury of the endothelium, migration of vascular smooth muscle cells (VSMC) to the tunica intima of the blood vessels, proliferation of vascular smooth muscle cells, accumulation of lipids within the cells (foam cells), and the like. In addition, under hypertensive conditions, which are associated with progression of arteriosclerosis, it is known that vascular cytoarchitecture is changed by responding to various loading factors to the vessels and remodeling of the vessels occurs. Remodeling of the vessels indicates structural changes in the vessels caused by hemodynamic changes such as changes of blood flow and tension of blood vessel walls. In addition to substances such as growth factors and cytokines, vasoactive substances are suggested to contribute to the development processes. For example, it is known that angiotensin II, which is an endothelium-derived vasoconstrictive factor, facilitates proliferation of vascular smooth muscle cells (Medical Clinics of Japan, Vol. 21, 1924, 1995), and also facilitates remodeling of vessels (Journal of Clinical and Experimental Medicine (IGAKU NO AYUMI), Vol. 193, 361, 2000).

However, detailed mechanisms of progression of arteriosclerosis from pathogenesis to advanced disease are not sufficiently clarified. In addition, detailed mechanisms of vascular remodeling are also unknown Although there are some reports describing relationships between angiotensin II receptor antagonists and vascular remodeling (Circulation, 104, 2716, 2001), the effects of calcium channel blockers on pathological changes in arteriosclerosis and vascular damage as well as their mechanisms are little known. Furthermore, the prophylactic and therapeutic effects of combined administration of a calcium channel blocker and an angiotensin II receptor antagonist against arteriosclerosis are little reported, if at all. Particularly, the effects of calcium channel blockers on the renin-angiotensin system and the synergistic effects of a calcium channel blocker and an angiotensin II receptor antagonist are little known, despite the fact that they are important subjects in therapeutic aspects of arteriosclerosis.

Furthermore, since percutaneous coronary intervention (PCI) including percutaneous transluminal coronary angioplasty (PTCA) and stent implantation have low invasiveness, they occupy the central position in current therapeutic strategies against ischemic heart diseases. However, restenosis appearing within several months after surgery in 30-45 % patients undergoing these surgical procedures is a major problem. As for the mechanisms of restenosis following PCI, decreases in the diameters of whole vessels in the late period after PCI (that is, remodeling) are considered important, in addition to hyperplasia and hypertrophy of neointima caused by proliferation of smooth muscle cells and accumulation of extracellular matrix, which is produced by the smooth muscle cells (Coronary Intervention, Vol. 1, 12; Medical Clinics of Japan, Vol. 21, 1924, 1995). Under these circumstances, development of new medicaments that can effectively prevent restenosis of vessels following PCI is needed. Nevertheless, no medicaments with high efficacy have so far been developed.

### [Disclosure of the invention]

The subject of the present invention is to provide medicaments for the prevention and/or treatment of arteriosclerosis. More concretely, it is the subject of the present invention to provide medicaments to prevent or to inhibit the proliferation of vascular smooth muscles and neointima formation in blood vessels. Furthermore, another subject of the present invention is to provide medicaments that effectively inhibit remodeling of vessels and prevent progression of arteriosclerosis as well as restenosis of vessels following PCI.

Furthermore, the other subject of the present invention is to provide medicaments for the prophylaxis or treatment of hypertension or diseases caused by hypertension. More concretely, the subject is to provide medicaments for the prophylaxis and/or medical treatment of hypertension, heart diseases [angina pectoris, myocardial infarction, arrhythmia (including sudden death), cardiac failure or cardiac hypertrophy], renal diseases (diabetic nephropathy, glomerulonephritis or nephrosclerosis) or cerebrovascular diseases (cerebral infarction or cerebral hemorrhage) (particularly medicaments for the prevention or treatment of hypertension).

The present inventors have fastidiously studied the subjects described above, and found that combined administration of a specific calcium channel blocker and a specific angiotensin II receptor antagonist potently prevents proliferation of vascular smooth muscle cells as well as neointima formation in blood vessels, and that the inhibitory action of the combined administration of the two kinds of agents was discovered to be synergistic, and also found that the inhibitory action was potently observed at lower doses than their effective doses when they were administered alone. Moreover, the present inventors found that combined administration as described above remarkably prevented vascular remodeling and that the medicament effectively inhibited restenosis following PCI.

Furthermore, the present inventors found that combined administration of the specific calcium channel blocker and the specific angiotensin II receptor antagonist described above could achieve excellent antihypertensive action. In addition, the present inventors found that the present medicament is remarkably effective for the prophylaxis and/or treatment of hypertension, heart diseases [angina pectoris, myocardial infarction, arrhythmia (including sudden death), cardiac failure or cardiac hypertrophy], renal diseases (diabetic nephropathy, glomerulonephritis or nephrosclerosis) or cerebrovascular disorders (cerebral infarction or cerebral hemorrhage). Thus the present invention was completed based on these findings described above.

The present invention provides a medicament for the prevention and/or treatment of arteriosclerosis, comprising the following composition:
(A) an angiotensin II receptor antagonist selected from the group consisting of a compound having a general formula (I), 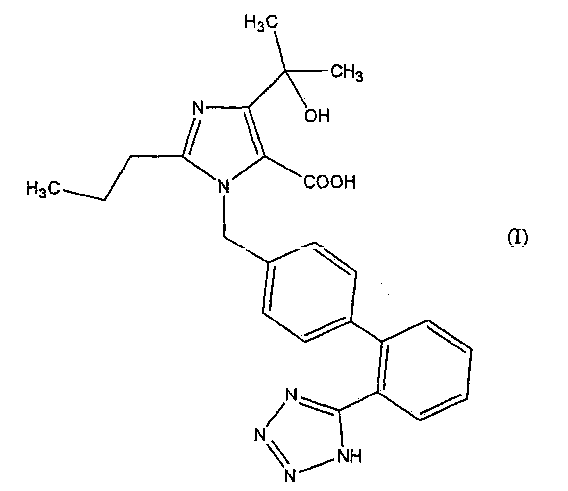 pharmacologically acceptable esters thereof and pharmacologically acceptable salts thereof; and
(B) a calcium channel blocker selected from the group consisting of 1,4-dihydropyridine derivatives and pharmacologically acceptable salts thereof as active ingredients.

Furthermore, from another different point of view of the present invention, it provides a medicament for the inhibition of the proliferation of vascular smooth muscle cells comprising the compound (A) and the compound (B) as active ingredients; a medicament for the inhibition of neointima formation in blood vessels comprising the compound (A) and the compound (B) as active ingredients; and a medicament for the inhibition of vascular remodeling comprising the compound (A) and the compound (B) as the active ingredients. These medicaments can be used, for example, as a prophylactic agent for restenosis following percutaneous coronary intervention. From this point of view, a medicament for the prevention of restenosis following percutaneous coronary intervention comprising the compound (A) and the compound (B) is provided by the present invention.

Furthermore, the present invention provides a medicament for the prevention and/or treatment of hypertension or diseases caused by hypertension comprising the following compounds as active ingredients;
(A) an angiotensin II receptor antagonist selected from the group consisting of a compound having the formula (I) described above, pharmacologically acceptable esters thereof and pharmacologically acceptable salts thereof; and
(B) a calcium channel blocker selected from the group consisting of 1,4-dihydropyridine derivatives and pharmacologically acceptable salts thereof; and a medicament for the prevention and/or treatment of heart diseases [angina pectoris, myocardial infarction, arrhythmia (including sudden death), cardiac failure, cardiac hypertrophy and the like], renal diseases (diabetic nephropathy, glomerulonephritis, nephrosclerosis and the like), or cerebrovascular disorders (cerebral infarction, cerebral hemorrhage and the like comprising the following compounds as active ingredients;

(A) an angiotensin II receptor antagonist selected from the group consisting of a compound having the formula (I) described above, pharmacologically acceptable esters thereof and pharmacologically acceptable salts thereof; and
(B) a calcium channel blocker selected from the group consisting of 1,4-dihydropyridine derivatives and pharmacologically acceptable salts thereof.

According to a preferred embodiment of the invention, the medicament described above is provided as a pharmaceutical composition comprising the compound (A) and the compound (B) as active ingredients. This pharmaceutical composition may contain one or more excipients for formulation. According to another preferred embodiment of the invention, a medicament described above to administer the compound (A) and the compound (B) at the same time or separately at certain intervals is provided.

Furthermore, according to a more preferred embodiment of the invention, the medicament described above is provided as a pharmaceutical composition comprising an angiotensin II receptor antagonist and a calcium channel blocker, wherein said angiotensin II receptor antagonist is (5-methyl-2-oxo 1,3-dioxolen-4-yl)methyl 4-(1-hydroxy-1-methylethyl)-2-propyl-1-[[2'-(1H-tetrazol-5-yl)biphenyl-4-yl]methyl]imidazol-5-carboxylate (hereinafter it is referred to as "olmesartan medoxomil" in some parts of the present specification) and said calcium channel blocker is any one selected from the group of calcium channel blockers comprising (±)-2-amino-1,4-dihydro-6-methyl-4-(3-nitrophenyl)-3,5-pyridine-dicarboxylate-3-(1-diphenylmethylazetidin-3-yl)ester 5-isopropylester (hereinafter it is referred to as "azelnidipine" in some parts of the present specification); amlodipine, benidipine, nitrendipine, manidipine, nicardipine, nifedipine, nisoldipine, cilnidipine, lercanidipine, niguldipine, nimodipine, aranidipine, efonidipine, bamidipine, felodipine, and nilvadipine; and the preferred calcium channel blocker is azelnidipine.

From another aspect of the present invention, the present invention provides the use of an angiotensin II receptor antagonist selected from the group consisting of a compound having the formula (I) described above, pharmacologically acceptable esters thereof and pharmacologically acceptable salts thereof to manufacture the medicament described above; and the use of a calcium channel blocker selected from the group consisting of 1,4-dihydropyridine derivatives and pharmacologically acceptable salts thereof to manufacture the medicament described above.

Furthermore, the present invention provides methods for the prophylaxis and/or treatment of arteriosclerosis, comprising any process of administration of effective doses of said compound (A) and said compound (B) to mammals including humans; methods for the inhibition of the proliferation of vascular smooth muscle cells, comprising any process of administration of effective doses of said compound (A) and said compound (B) to mammals including humans; methods for the inhibition of neointima formation of blood vessels, comprising any process of administration of effective doses of said compound (A) and said compound (B) to mammals including humans; methods for the inhibition of vascular remodeling, comprising any process of administration of effective doses of said compound (A) and said compound (B) to mammals including humans; and methods for the inhibition of restenosis following percutaneous coronary intervention, comprising any process of administration of effective doses of said compound (A) and said compound (B) to mammals including humans. Preferably, in the present invention the effective dose of each composition comprising the compound (A) and the compound (B) is around the lowest limit or below the lowest limit of the effective dose of the compound (A) or the compound (B) when administered alone.

Furthermore, the present invention provides methods for the prophylaxis and/or treatment of hypertension or diseases caused by hypertension, comprising any process of administration of effective doses of said compound (A) and said compound (B) to mammals including humans; methods for the prophylaxis or treatment of hypertension, comprising any process of administration of effective doses of said compound (A) and said compound (B) to mammals including humans; methods for the prophylaxis or treatment of heart diseases, comprising any process of administration of effective doses of said compound (A) and said compound (B) to mammals including humans; methods for the prophylaxis or treatment of angina pectoris, comprising any process of administration of effective doses of said compound (A) and said compound (B) to mammals including humans; methods for the prophylaxis or treatment of myocardial infarction, comprising any process of administration of effective doses of said compound (A) and said compound (B) to mammals including humans; methods for the prophylaxis or treatment of arrhythmia, comprising any process of administration of effective doses of said compound (A) and said compound (B) to mammals including humans; methods for the prophylaxis of sudden death, comprising any process of administration of effective doses of said compound (A) and said compound (B) to mammals including humans; methods for the prophylaxis or treatment of heart failure, comprising any process of administration of effective doses of said compound (A) and said compound (B) to mammals including humans; methods for the prophylaxis or treatment of cardiac hypertrophy, comprising any process of administration of effective doses of said compound (A) and said compound (B) to mammals including humans; methods for the prophylaxis or treatment of renal diseases, comprising any process of administration of effective doses of said compound (A) and said compound (B) to mammals including humans; methods for the prophylaxis or treatment of diabetic nephropathy, comprising any process of administration of effective doses of said compound (A) and said compound (B) to mammals including humans; methods for the prophylaxis or treatment of glomerulonephritis, comprising any process of administration of effective doses of said compound (A) and said compound (B) to mammals including humans; methods for the prophylaxis or treatment of nephrosclerosis, comprising any process of administration of effective doses of said compound (A) and said compound (B) to mammals including humans; methods for the prophylaxis or treatment of cerebrovascular diseases, comprising any process of administration of effective doses of said compound (A) and said compound (B) to mammals including humans; methods for the prophylaxis or treatment of cerebral infarction, comprising any process of administration of effective doses of said compound (A) and said compound (B) to mammals including humans; and/or methods for the prophylaxis or treatment of cerebral haemorrhage, comprising any process of administration of effective doses of said compound (A) and said compound (B) to mammals including humans.

### [Brief description of the drawings]

Fig. 1 indicates the results for the inhibition of DNA synthesis in vascular smooth muscle cells by a calcium channel blocker, azelnidipine, at a dose range of 0.1 to 1.0 mg/kg/day.
Fig. 2 shows the results for inhibition of neointima formation in blood vessels by a calcium channel blocker, azelnidipine, at a dose range of 0.1 to 1.0 mg/kg/day.
Fig. 3 represents the results for inhibition of DNA synthesis in vascular smooth muscle cells by an angiotensin II receptor antagonist, olmesartan, at a dose range of 0.5 to 3.0 mg/kg/day.
Fig. 4 indicates the results for inhibition of neointima formation in blood vessels by an angiotensin II receptor antagonist, olmesartan, at a dose range of 0.5 to 3.0 mg/kg/day.
Fig. 5 shows the results for inhibition of DNA synthesis in vascular smooth muscle cells by simultaneous administration of azelnidipine and olmesartan at doses of 0.1 mg/kg/day and 0.5 mg/kg/day, respectively (at which doses they did not elicit any significant effects by each of these drugs alone).
Fig. 6 represents the results for inhibition of neointima formation in blood vessels by simultaneous administration of azelnidipine and olmesartan at doses of 0.1 mg/kg/day and 0.5 mg/kg/day, respectively (at which doses they did not elicit any significant effects by each of these drugs alone).
Fig. 7 indicates the results for inhibition of potentiation of DNA synthesis in cultured rat vascular smooth muscle cells following stimulation of angiotensin II receptors by azelnidipine in a concentration-dependent manner.
Fig. 8 shows the results for significant inhibition of DNA synthesis in cultured vascular smooth muscle cells by co-administration of azelnidipine and olmesartan at low concentrations, at which concentrations they did not elicit any significant effects by each of these drugs alone.

### [Best mode for carrying out the invention]

The medicaments of the present invention are characterized by containing (A) an angiotensin II receptor antagonist selected from the group consisting of a compound having the formula (I) described above, pharmacologically acceptable esters thereof and pharmacologically acceptable salts thereof; and (B) a calcium channel blocker selected from the group consisting of 1,4-dihydropyridine derivatives and pharmacologically acceptable salts thereof as active ingredients.

The compound having the formula (I) described above, [4-(1-hydroxy-1-methylethyl)-2-propyl-1-[[2'-(1H-tetrazol-5-yl)biphenyl-4-yl]methyl]imidazol-5-carboxylic acid] is a known compound, and for example, it is easily obtained by methods disclosed in Japanese Patent Publication (Kokai) Number Hei 5-78328 (USP 5,616,599 Specification), and the like. The pharmacologically acceptable salts of the compound having the formula (I) described above are not specifically restricted and these salts can be selected by a person with an ordinary skill in the art. As pharmacologically acceptable salts of the compound having the formula (I) described above, such salts are, for example, an alkaline metal salt such as sodium salt, potassium salt or lithium salt; an alkaline earth metal salt such as calcium salt or magnesium salt; a metal salt such as aluminium salt, iron salt, zinc salt, copper salt, nickel salt or cobalt salt; an amine salt such as an ammonium salt, t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt or tris(hydroxymethyl)aminomethane salt, but not restricted to these salts. Preferably alkaline metal salts can be used, and particularly preferably the sodium salt can be used.

The pharmacologically acceptable esters of the compound having the formula (I) comprise the compound having the formula (I) of which a carboxyl moiety is esterified. The pharmacologically acceptable esters are not particularly restricted, and can be selected by a person with an ordinary skill in the art. In the case of said esters, it is preferable that such esters can be cleaved by a biological process such as hydrolysis *in vivo.* The group constituting the said esters (the group shown as R when the esters thereof are expressed as -COOR) can be, for example, a C₁-C₄ alkoxy C₁-C₄ alkyl group such as methoxyethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, propoxymethyl, isopropoxymethyl, butoxymethyl or t-butoxymethyl; a C₁-C₄ alkoxylated C₁-C₄ alkoxy C₁-C₄ alkyl group such as 2-methoxyethoxymethyl; a C₆-C₁₀ aryloxy C₁-C₄ alkyl group such as phenoxymethyl; a halogenated C₁-C₄ alkoxy C₁-C₄ alkyl group such as 2,2,2-trichloroethoxymethyl or bis(2-chloroethoxy)methyl; a C₁-C₄ alkoxycarbonyl C₁-C₄ alkyl group such as methoxycarbonylmethyl; a cyano C₁-C₄ alkyl group such as cyanomethyl or 2-cyanoethyl; a C₁-C₄ alkylthiomethyl group such as methylthiomethyl or ethylthiomethyl; a C₆-C₁₀ arylthiomethyl group such as phenylthiomethyl or naphthylthiomethyl; a C₁-C₄ alkylsulfonyl C₁-C₄ lower alkyl group, which may be optionally substituted with a halogen atom(s) such as 2-methanesulfonylethyl or 2-trifluoromethanesulfonylethyl; a C₆-C₁₀ arylsulfonyl C₁-C₄ alkyl group such as 2-benzenesulfonylethyl or 2-toluenesulfonylethyl; a C₁-C₇ aliphatic acyloxy C₁-C₄ alkyl group such as formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl or 1-pivaloyloxyhexyl; a C₅-C₆ cycloalkylcarbonyloxy C₁-C₄ alkyl group such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl or 1-cyclohexylcarbonyloxybutyl; a C₆-C₁₀ arylcarbonyloxy C₁-C₄ alkyl group such as benzoyloxymethyl; a C₁-C₆ alkoxycarbonyloxy C₁-C₄ alkyl group such as methoxycarbonyloxymethyl, 1-(methoxycarbonyloxy)ethyl, 1-(methoxycarbonyloxy)propyl, 1-(methoxycarbonyloxy)butyl 1-(methoxycarbonyloxy)pentyl, 1-(methoxycarbonyloxy)hexyl, ethoxycarbonyloxymethyl, 1-(ethoxycarbonyloxy)ethyl, 1-(ethoxycarbonyloxy)propyl, 1-(ethoxycarbonyloxy)butyl, 1-(ethoxycarbonyloxy)pentyl, 1-(ethoxycarbonyloxy)hexyl, propoxycarbonyloxymethyl, 1-(propoxycarbonyloxy)ethyl, 1-(propoxycarbonyloxy)propyl, 1-(propoxycarbonyloxy)butyl, isopropoxycarbonyloxymethyl, 1-(isopropoxycarbonyloxy)ethyl, 1-(isopropoxycarbonyloxy)butyl, butoxycarbonyloxymethyl, 1-(butoxycarbonyloxy)ethyl, 1-(butoxycarbonyloxy)propyl, 1-(butoxycarbonyloxy)butyl, isobutoxycarbonyloxymethyl, 1-(isobutoxycarbonyloxy)ethyl, 1-(isobutoxycarbonyloxy)propyl, 1-(isobutoxycarbonyloxy)butyl, t-butoxycarbonyloxymethyl, 1-(t-butoxycarbonyloxy)ethyl, pentyloxycarbonyloxymethyl, 1-(pentyloxycarbonyloxy)ethyl, 1-(pentyloxycarbonyloxy)propyl, hexyloxycarbonyloxymethyl, 1-(hexyloxycarbonyloxy)ethyl or 1-(hexyloxycarbonyloxy)propyl; a C₅-C₆ cycloalkyloxycarbonyloxy C₁-C₄ alkyl group such as cyclopentyloxycarbonyloxymethyl, 1-(cyclopentyloxycarbonyloxy)ethyl, 1-(cyclopentyloxycarbonyloxy)propyl, 1-(cyclopentyloxycarbonyloxy)butyl, cyclohexyloxycarbonyloxymethyl, 1-(cyclohexyloxycarbonyloxy)ethyl, 1-(cyclohexyloxycarbonyloxy)propyl or 1-(cyclohexyloxycarbonyloxy)butyl; a [5-(C₁-C₄ alkyl)-2-oxo-1,3-dioxolen-4-yl]methyl group such as (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl or (5-butyl-2-oxo-1,3-dioxolen-4-yl)methy; a [5-(phenyl, which may be optionally substituted with a C₁-C₄ alkyl, C₁-C₄ alkoxy or halogen atom(s))-2-oxo-1,3-dioxolen-4-yl]methyl group such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl or [5-(4-chlorophenyl)-2-oxo-1,3-dioxolen-4-yl]methyl; or a phthalidyl group, which may be optionally substituted with a C₁-C₄ alkyl or C₁-C₄ alkoxy group(s), such as phthalidyl, dimethylphthalidyl or dimethoxyphthalidyl, and is preferably a pivaloyloxymethyl group, phthalidyl group or (5-methyl-2-oxo-1,3-dioxolen-4-yl)methyl group, and more preferably a (5-methyl-2-oxo-1,3-dioxolen-4-yl)methyl group.

In the case that the esters of the compound of formula (I) described above form pharmacologically acceptable salts, the pharmacologically acceptable salts can be selected by a person with an ordinary skill in the art, and are not particularly restricted. Such salts can be, for example, a hydrohalogenic acid salt such as a hydrofluoride, hydrochloride, hydrobromide or hydroiodide; a nitrate; a perchlorate; a sulfate; a phosphate; a C₁-C₄ alkanesulfonic acid salt; which may be optionally substituted with a halogen atom(s) such as a methanesulfonate, trifluoromethanesulfonate or ethanesulfonate; a C₆-C₁₀ arylsulfonic acid salt, which may be optionally substituted with a C₁-C₄ alkyl group(s), such as a benzenesulfonate or p-toluenesulfonate; a C₁-C₆ aliphatic acid salt such as an acetate, malate, fumarate, succinate, citrate, tartrate, oxalate or maleate; or an amino acid salt such as a glycine salt, lysine salt, arginine salt, ornithine salt, glutamic acid salt or aspartic acid salt, and is preferably a hydrochloride, nitrate, sulfate or phosphate, and is particularly preferably a hydrochloride.

The angiotensin II receptor antagonist, which is used as the compound (A), is preferably the compound having the formula (I) described above or a pharmacologically acceptable ester thereof, more preferably a pharmacologically acceptable ester of said compound having the formula (I), and further more preferably a pivaloyloxymethyl ester, phthalidyl ester or (5-methyl-2-oxo-1,3-dioxolen-4-yl)methyl ester of the compound having the formula (I). Most preferably, (5-methyl-2-oxo-1,3-dioxolen-4-yl)methyl 4-(1-hydroxy-1-methylethyl)-2-propyl-1-[[2'-(1H-tetrazol-5-yl)biphenyl-4-yl]methyl]imidazol-5-carboxylate can be used.

As the compound selected from the group consisting of a compound having the formula (I) described above, pharmacologically acceptable esters thereof and pharmacologically acceptable salts thereof, their hydrates or solvates can also be used. In the case that the pharmacologically acceptable esters of the compound having the formula (I) are used, some esterified compounds may have one or more asymmetric carbons, but optical isomers purified based on the said asymmetric carbons or stereoisomers such as diastereoisomers or any mixtures of these stereoisomers or racemates can also be used as the compound (A).

A calcium channel blocker including 1,4-dihydropyridine derivatives, which is used as the compound (B), is a calcium channel blocker characterized by having the 1,4-dihydropyridine moiety or chemically equivalent structural moiety to the 1,4-dihydropyridine moiety in the molecule. Many medicaments are proposed as calcium channel blockers including the 1,4-dihydropyridine derivatives and are actually used clinically, and a person with an ordinary skill in the art can select any suitable compounds exerting the effects of the present invention. As 1,4-dihydropyridine calcium channel blockers, for example, azelnidipine, amlodipine, benidipine, nitrendipine, manidipine, nicardipine, nifedipine, nisoldipine, cilnidipine, lercanidipine, niguldipine, nimodipine, aranidipine, efonidipine, barnidipine, felodipine, or nilvadipine can be used, but the scope of the present invention should not be limited to these calcium channel blockers exemplified. In addition, azelnidipine can be easily manufactured according to the methods disclosed in Japanese Patent Publication (Kokai) Number Sho 63-253082 (USP 4,772,596 Specification) and the like. Furthermore, amlodipine can be easily manufactured according to the methods disclosed in USP 4,572,909 Specification or USP 4,879,303 Specification.

Since the pharmacologically acceptable salts of 1,4-dihydropyridine derivatives are not specifically restricted, any salts thereof can be selected by a person with an ordinary skill in the art. The pharmacologically acceptable salts can be acid addition salts or base addition salts. These salts can be, for example, an alkaline metal salt such as a sodium salt, potassium salt or lithium salt; an alkaline earth metal salt such as a calcium salt or magnesium salt; a metal salt such as an aluminum salt, iron salt, zinc salt, copper salt, nickel salt or cobalt salt; or a base addition salt, for example, an amine salt such as an ammonium salt, t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt or tris(hydroxymethyl)aminomethane salt; or an acid addition salt, for example, a hydrohalide such as a hydrofluoride, hydrochloride, hydrobromide or hydroiodide; a nitrate; a perchlorate; a sulfate; a phosphate; a C₁-C₄ alkanesulfonate, which may be optionally substituted with a halogen atom(s) such as a methanesulfonate, trifluoromethanesulfonate or ethanesulfonate; a C₆-C₁₀ arylsulfonate, which may be optionally substituted with a C₁-C₄ alkyl group(s) such as a benzenesulfonate or p-toluenesulfonate; a C₁-C₆ aliphatic acid salt such as an acetate, malate, fumarate, succinate, citrate, tartrate, oxalate or maleate; or an amino acid salt such as a glycine salt, lysine salt, arginine salt, omithine salt, glutamic acid salt or aspartic acid salt, but the scope of the present invention should not be restricted to these salts described above.

As a calcium channel blocker including 1,4-dihydropyridine derivatives, hydrates or solvates of the compounds described above and pharmacologically acceptable salts thereof can be used. In addition, some calcium channel blockers including 1,4-dihydropyridine derivatives contain one or more asymmetric carbons in their molecules. In these cases, optical isomers purified based on the asymmetric carbons or stereoisomers such as diastereoisomers, or any mixtures of stereoisomers, or racemates can also be used as the compound (B). As the compound (B), (±)-2-amino-1,4-dihydro-6-methyl-4-(3-nitrophenyl)-3,5-pyridinedicarboxylic acid 3-(1-diphenylmethylazetidin-3-yl) ester 5-isopropyl ester, (R)-2-amino-1,4-dihydro-6-methyl-4-(3-nitrophenyl)-3,5-pyridinedicarboxylic acid 3-(1-diphenylmethylazetidin-3-yl) ester 5-isopropyl ester, amlodipine besylate, or amlodipine maleate is preferred.

As concretely shown in the Test Examples of the present specification, the medicament of the present invention consisting of the compound (A) and the compound (B) works synergistically and inhibits neointima formation of blood vessels and proliferation of vascular smooth muscle cells and, as a result, inhibits vascular remodeling. Based on the actions described above, the medicaments of the present invention can be used for the prophylaxis of restenosis following percutaneous coronary intervention in addition to the prophylaxis and/or treatment of arteriosclerosis.

The medicament of the present invention is characterized by exerting excellent inhibitory effects on neointima formation of blood vessels and proliferation of vascular smooth muscle cells due to combined administration of the compound (A) and the compound (B) at their lowest limit doses or lower than their lowest limit doses of each composition administered alone. Particularly, it is preferable to co-administer the compound (A) and the compound (B) at low doses at which no effects are elicited on administration of each composition alone.

As concretely shown in Test Examples of the present specification, the medicament of the present invention lowers blood pressure more effectively by synergistic action of the compound (A) and the compound (B). Based on these actions described above, the medicament of the present invention can be used for the prophylaxis and/or treatment of hypertension, heart diseases (angina pectoris, myocardial infarction, arrhythmia (including sudden death), cardiac failure, cardiac hypertrophy and the like), renal diseases (diabetic nephropathy, glomerulonephritis, nephrosclerosis and the like) or cerebrovascular disorders (cerebral infarction, cerebral hemorrhage and the like), and preferably for the treatment. The medicament of the present invention comprising an angiotensin II receptor antagonist and a calcium channel blocker exerts more excellent effects by combined administration of an angiotensin II receptor antagonist and a calcium channel blocker than either one of these agents administered alone.

The medicament of the present invention may be prepared as a pharmaceutical composition (so-called as a mode of "combination drug") comprising the compound (A) and the compound (B) as the active ingredients. For example, each active ingredient may be mixed together and may be prepared as a physically single formulation. In addition, the compound (A) and the compound (B) may be prepared separately as an independent formulation and can be provided as a medicament containing a combination of each mode of formulation. The latter medicament can be used as a medicament to administer the compound (A) and the compound (B) at the same time or separately at certain intervals.

Administration of the compound (A) and the compound (B) "at the same time" described in the present specification includes administration of the compound (A) and the compound (B) roughly at the same time and not just restricted to exactly the same time. There is no restriction of the dosage form for administration at the same time; for example, it is included that either one of the compositions is orally administered and the other composition is non-orally administered. Nevertheless, it is favourable to prepare the invention as a single pharmaceutical composition and to take the both compositions simultaneously.

Independent administration of the compound (A) and the compound (B) "at certain intervals" described in the present specification means that the compound (A) and the compound (B) described in the present invention are to be taken independently at different times. The mode of administration for separate administration at certain intervals has no restriction. For instance, it includes that first an angiotensin II receptor antagonist is administered, and then after a certain interval, a calcium channel blocker is administered, or first a calcium channel blocker is administered, and then after a certain interval, an angiotensin II receptor antagonist is administered, but the dosage form has no restriction.

The present medicament is manufactured by previously known methods in a suitable dosage form, such as tablets, capsules, granules, powders or syrups for oral administration, or injections or suppositories for parenteral administration, by using pharmacologically acceptable and suitable additive agents such as excipients, lubricants, binders, disintegrants, demulsifiers, stabilizers, flavours, diluents, and the like, if necessary, in addition to the compound (A) and the compound (B), which are the active ingredients, and can be administered. Since the compound (A) and the compound (B) containing in the medicament of the present invention are compounds to be orally administered in general, the medicament of the present invention is favourable to be orally administered.

As "excipients", for instance, organic excipients including sugar derivatives such as lactose, sucrose, glucose, mannitol or sorbitol; starch derivatives such as corn starch, potato starch, α-starch or dextrin; cellulose derivatives such as crystalline cellulose; gum arabic; dextran; or pullulan; and inorganic excipients including silicate derivatives such as light anhydrous silicic acid, synthetic aluminium silicate, calcium silicate or magnesium aluminometasilicate; phosphates such as calcium hydrogenphosphate; carbonates such as calcium carbonate; or sulfates such as calcium sulfate can be listed.

As "lubricants", for instance, stearic acid; metal salts of stearic acid such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; laurylsulfates such as sodium lauryl sulfate or magnesium lauryl sulfate; silicates such as silicic anhydride and silicic hydrate; or the starch derivatives described above can be listed.

As "binders", for instance, hydroxypropylcellulose, hydroxypropylmethylcellulose, polyvinylpyrrolidone, macrogol, or similar excipients to those described above can be listed.

As "disintegrants", for instance, cellulose derivatives such as low-substituted hydroxypropylcellulose, carboxymethylcellulose, calcium carboxymethylcellulose or internally crosslinked sodium carboxymethylcellulose; and chemically modified starch/cellulose derivatives such as carboxymethylstarch or sodium carboxymethylstarch can be listed.

As "demulsifiers", for instance, colloidal clay such as bentonite or veegum; metal hydroxides such as magnesium hydroxide or aluminium hydroxide; anionic surfactants such as sodium lauryl sulfate or calcium stearate; cationic surfactants such as benzalkonium chloride; or nonionic surfactants such as polyoxyethylenealkylether, polyoxyethylene sorbitan fatty acid ester or sucrose esters of fatty acids can be listed.

As "stabilizers", for instance, p-hydroxybenzoate esters such as methylparaben or propylparaben; alcohols such as chlorobutanol, benzyl alcohol or phenylethyl alcohol; benzalkonium chloride; phenols such as phenol or cresol; thimerosal; dehydroacetic acid; or sorbic acid can be listed.

As "flavours", for instance, sweeteners such as saccharin sodium or aspartame; acidifiers such as citric acid, malic acid or tartaric acid; or flavours such as menthol, lemon or orange can be listed.

As "diluents", conventionally used diluents, for instance, lactose, mannitol, glucose, sucrose, calcium sulfate, calcium phosphate, hydroxypropylcellulose, microcrystalline cellulose, water, ethanol, polyethyleneglycol, propyleneglycol, glycerol, starch, polyvinylpyrrolidone, magnesium aluminometasilicate or a mixture of these compounds can be listed.

Doses of an angiotensin II receptor antagonist and a calcium channel blocker, which are active ingredients, and their dosing ratio can be selected in a suitable manner depending on various factors such as the drugs' activities and symptoms, age and body weight of the patients. Although the dosage varies depending on symptoms, age and the like, in the case of oral administration, 0.1 mg (preferably 0.5 mg) as a lower limit and 1000 mg (preferably 500 mg) as an upper limit per one time for a human adult and one to six times per day depending on the symptoms of the patients can be administered, at the same time or separately at certain intervals. In the case of parenteral administration, 0.01 mg (preferably 0.05 mg) as a lower limit and 100 mg (preferably 50 mg) as an upper limit per one time for a human adult and one to six times per day depending on the symptoms of the patients, at the same time or separately at certain intervals, can be administered. For instance, the dosing ratio of the compound (A) and the compound (B) can range from 1:10000 to 10000:1 in weight ratios, preferably it can be in the range of 1:1000 to 1000:1, and more preferably it can be in the range of 1:100 to 100:1.

When the medicament of the present invention is used for the prophylaxis and/or treatment of arteriosclerosis, it is desirable, in general, that the blood concentration of the compound (A) and the compound (B) after administration is properly adjusted so as to be around the lowest limit or below the lowest limit of the compound (A) or the compound (B) when administered alone.

When the medicament of the present invention is used for the prophylaxis and/or treatment of hypertension, the dosage of angiotensin II receptor antagonist can be prescribed at lower doses than the dosage of the angiotensin II receptor antagonist when the angiotensin II receptor antagonist is used alone as a hypotensive agent, which is its original use, and the dosage of the angiotensin II receptor antagonist can be enormously reduced, because excellent antihypertensive action can be achieved by combined administration of an angiotensin II receptor antagonist with a calcium channel blocker.

### [Examples]

The present invention will be hereinafter described in more detail by way of the Examples and Test Examples described below, but the scope of the present invention should not be limited to these examples. In the Test Examples, "olmesartan medoxomil" is simply called "olmesartan".

### Test Example 1: Inhibitory effects against arteriosclerosis

### (A) Materials and Methods

(1) Cuff-induced vascular injury model
   C57BL/6 mice aged 10 weeks were used. In a part of this study, AT1a receptor gene knock out (AT1aKO) mice were also used. Inflammatory vascular damage was induced in mice by loosely placing a polyethylene tube which was cut longitudinally to open the tube, around the femoral artery of mice. In the damaged artery, the following observations were determined. The usefulness of this model of vascular damage to analyze vascular remodeling has been previously reported (Physiol. Genomics., 2, pp. 13 - 30, 2000; Circulation, 104, pp. 2716 - 2721, 2001; Circulation, 106, pp. 847 - 853, 2002).
(2) Neointima formation in the blood vessels and DNA synthesis
   A paraffin-embedded section of the damaged artery was prepared 14 days after cuff placement, Elastica van Gieson staining was carried out, and the cross-sectional area of the neointima and tunica media of the blood vessels was determined by image analysis software. For quantification of DNA synthesis, bromodeoxyuridine (BrdU) was injected into the mice 7 days after cuff placement and BrdU index calculated from incorporation into the nuclei of the cells was calculated.
(3) Olmesartan, an AT1 receptor blocker, was intraperitoneally injected into wild-type mice by osmotic minipump, and the dose-dependency of olmesartan was investigated as described in (2). Oral administration of azelnidipine to the wild-type mice was started after cuff placement, and dose-dependency of azelnidipine was investigated as described in (2).
(4) Both olmesartan and azelnidipine were simultaneously administered to wild-type mice, and the effects of co-administration of olmesartan and azelnidipine were compared with those of administration of either olmesartan or azelnidipine alone as described in (2). Effects of olmesartan and azelnidipine were investigated at effective doses of either olmesartan or azelnidipine alone and at insufficient doses to elicit significant effects by either olmesartan or azelnidipine alone, so as to determine synergistic effects of these two agents.
(5) By using cultured rat vascular smooth muscle cells, the effects of co-treatment of olmesartan and azelnidipine on facilitation of DNA synthesis following stimulation by angiotensin II (determined by incorporation of [³H]thymidine) were investigated.

### (B) Results

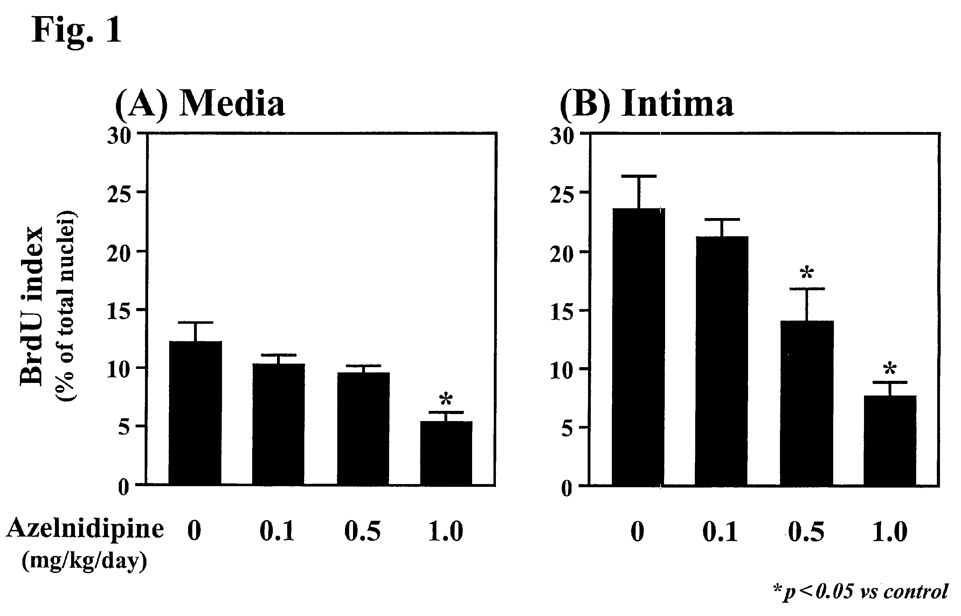

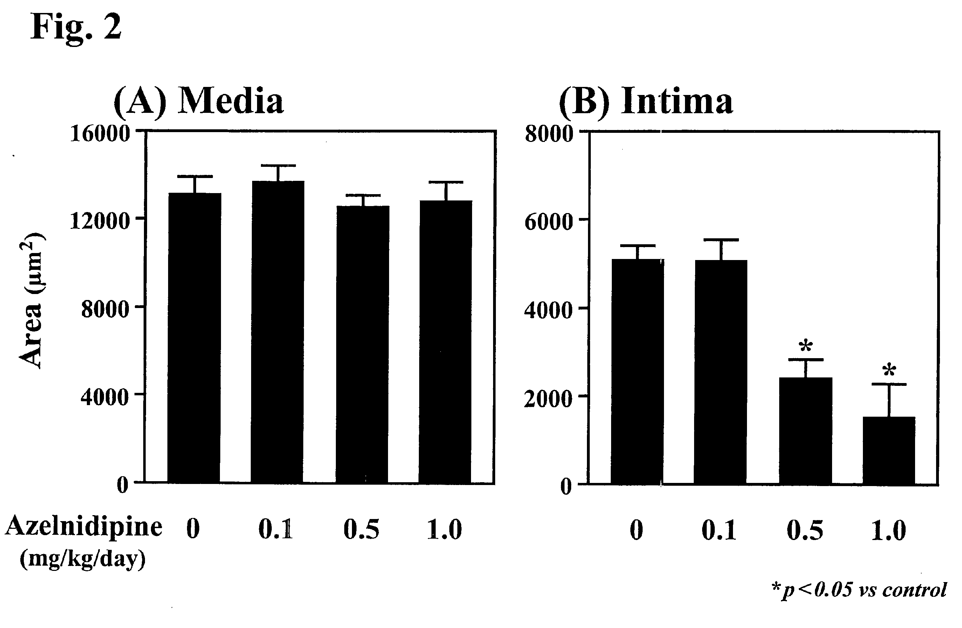

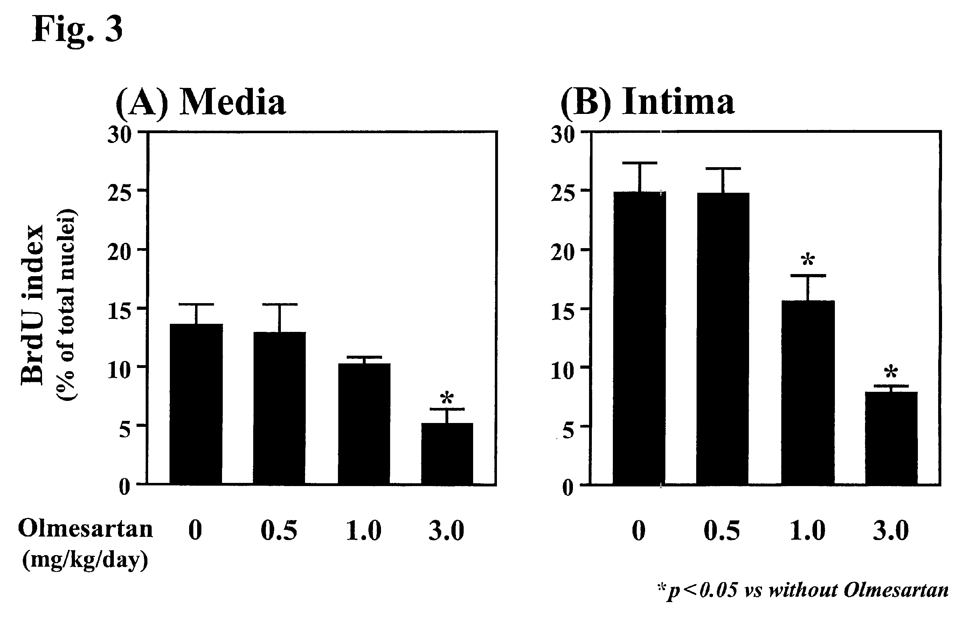

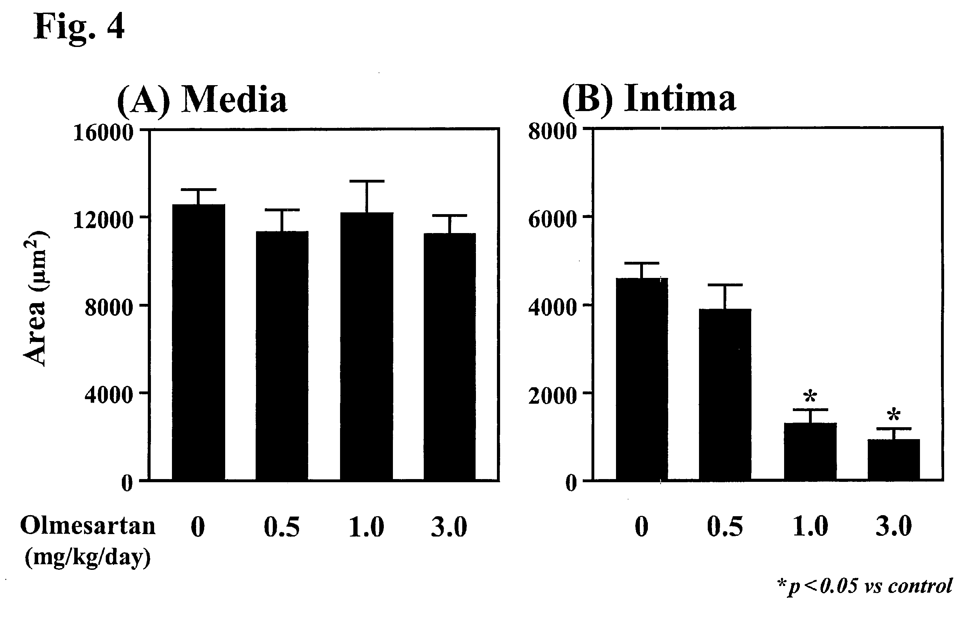

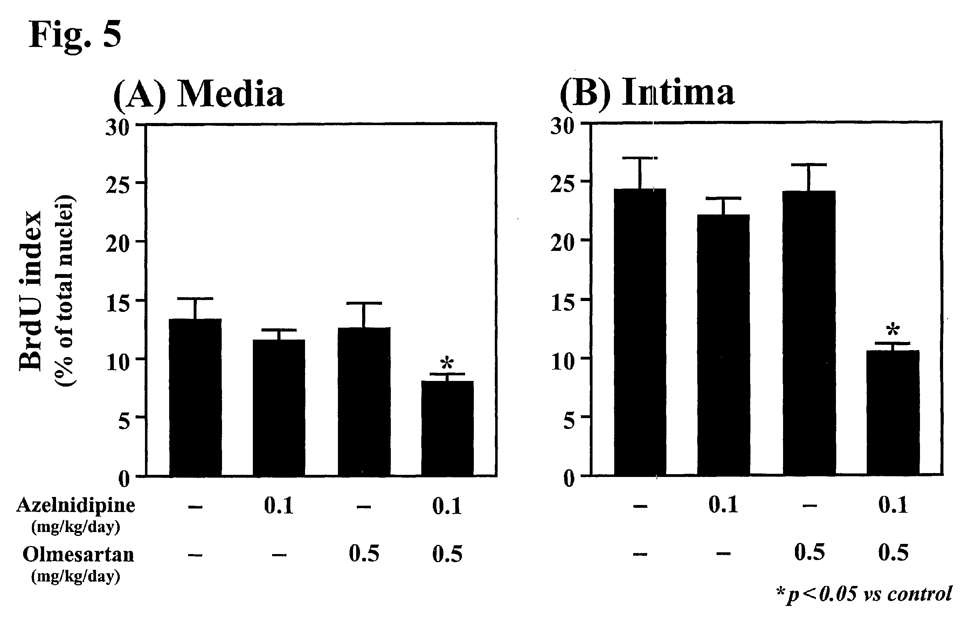

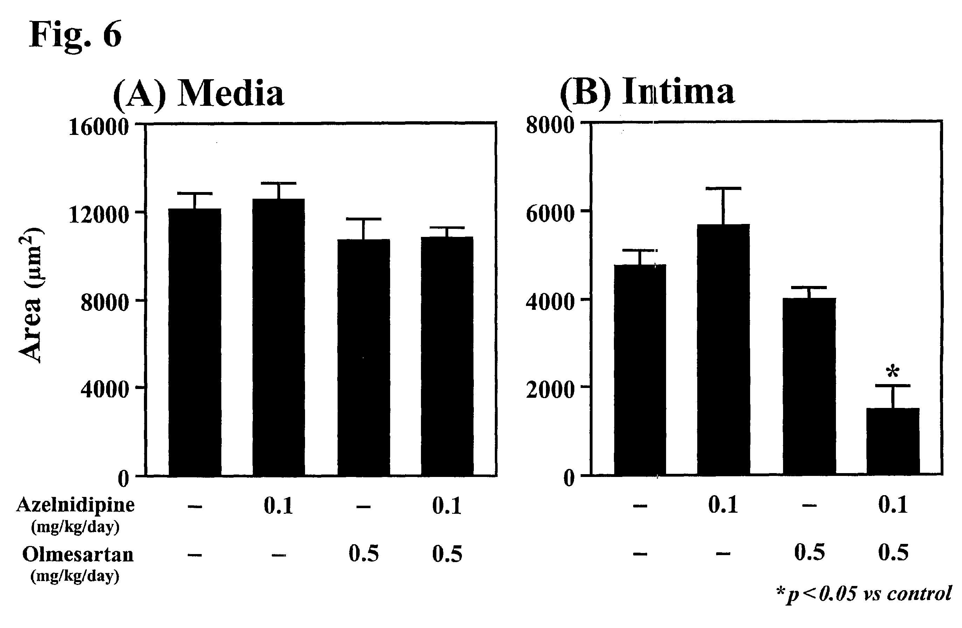

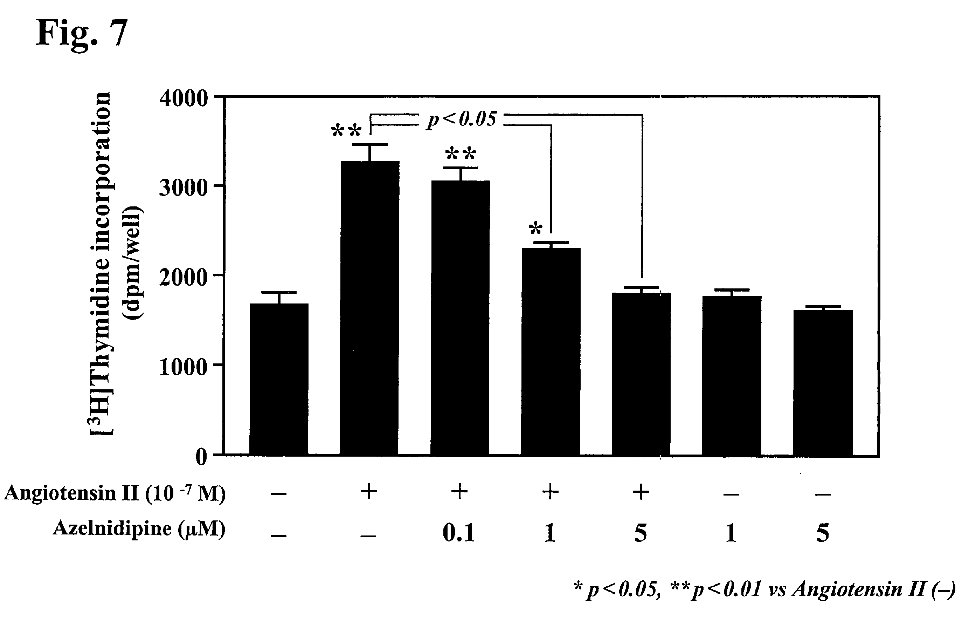

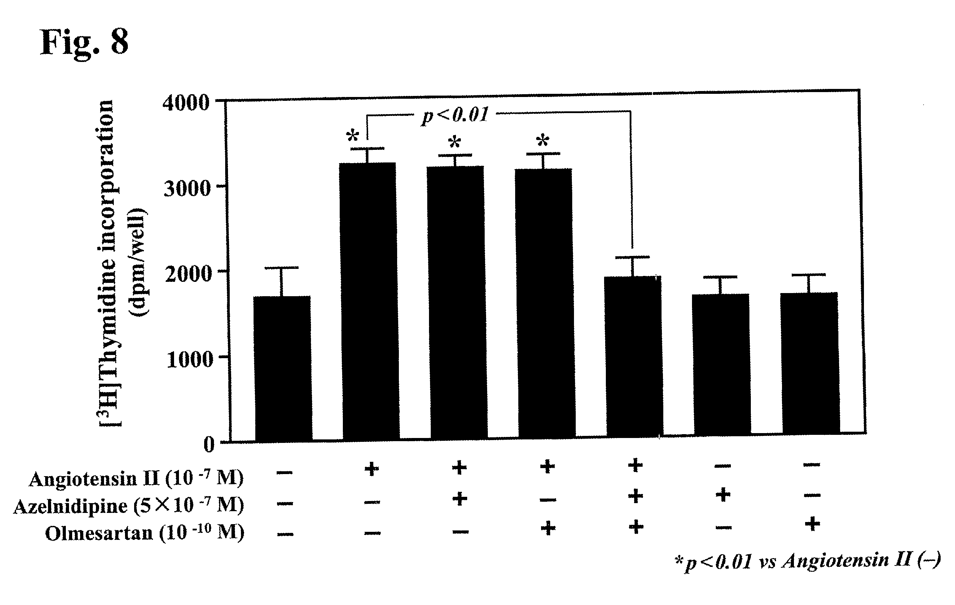

(1) In the cuff-induced vascular damage model of wild-type mice, DNA synthesis of vascular smooth muscle cells was increased and neointima formation in the blood vessels was potentiated. These changes were inhibited by azelnidipine in a dose-dependent manner at a dose range of 0.1 to 1.0 mg/kg/day, without any effects on blood pressure (Fig. 1 and Fig. 2). In addition, olmesartan exhibited similar inhibitory effects in a dose-dependent manner at a dose range of 0.5 to 3.0 mg/kg/day, without any effects on blood pressure (Fig. 3 and Fig. 4). When 0.1 mg/kg/day of azelnidipine and 0.5 mg/kg/day of olmesartan (both of these drugs did not elicit any significant effects at these doses) were administered at the same time, the increase in DNA synthesis of vascular smooth muscle cells and potentiation of neointima formation in the blood vessels were significantly suppressed (Fig. 5 and Fig. 6). From these results, it was clearly demonstrated in vivo that co-administration of azelnidipine and olmesartan works synergistically, inhibits proliferation of the vascular smooth muscle cells and improves vascular remodeling.
(2) The synergistic action of azelnidipine and olmesartan described above was investigated in an in vitro study. As shown in Fig. 7, facilitation of DNA synthesis in cultured rat vascular smooth muscle cells following stimulation by angiotensin II was suppressed by administration of azelnidipine in a concentration-dependent manner. When low doses of azelnidipine and olmesartan insufficient to elicit any effects alone were co-administered, DNA synthesis of cultured rat vascular smooth muscle cells was significantly suppressed (Fig. 8).

### Test Example 2: Antihypertensive activity

Surgical operations were carried out in 56 spontaneously hypertensive rats (SHRs, SPF grade, Breeder: Hoshino Laboratory Animals) aged 20 weeks to implant transmitters for recording their blood pressures. After recovery from the surgical operations, their blood pressure was monitored starting at 24 weeks of age. 0.5% carboxymethylcellulose sodium (CMC-Na) solution (2 ml/kg) was orally administered for 7 successive days (once daily) by a dosing cannula. The animals were divided into 7 groups (8 rats per group) with homogenous blood pressure in each group based on their blood pressure determined on the 5th and 6th days from initiation of blood pressure monitoring (the constitution of each group is illustrated in Table 1). Then either 0.5% CMC-Na solution (2 ml/kg: control group) or test drug solution (2 ml/kg) in which the test substance was suspended in 0.5% CMC-Na solution was administered from 25 weeks of age for 14 successive days (once daily) and changes in blood pressure were observed. Changes in the blood pressure of group 6 and group 7 are shown in Table 2. (Values in the tables represent mean ± S.D.) Excellent antihypertensive effects were observed in animals in the group co-administered olmesartan plus azelnidipine.

**Table 1.**

| | |
|---|---|
| Group 1 | Control group (0.5% CMC-Na solution) |
| Group 2 | Olmesartan medoxomil (0.2 mg/kg) |
| Group 3 | Olmesartan medoxomil (1.0 mg/kg) |
| Group 4 | Azelnidipine (2.0 mg/kg) |
| Group 5 | Azelnidipine (5.0 mg/kg) |
| Group 6 | Olmesartan medoxomil (0.2 mg/kg) + azelnidipine (2.0 mg/kg) |
| Group 7 | Olmesartan medoxomil (1.0 mg/kg) + azelnidipine (5.0 mg/kg) |

**Table 2**

| Test Substance | n | Blood Pressure (mmHg) | | | |
|---|---|---|---|---|---|
| | | 1st Day | 2nd Day | 5th Day | 13th Day |
| Control Group | 8 | 169.2 ± 23.8 | 167.0 ± 20.6 | 170.4 ± 21.1 | 173.0 ± 21.2 |
| Group 6 | 8 | 150.3 ± 11.7 | 144.5 ± 9.8 | 147.2 ± 10.1 | 145.9 ± 8.8 |
| Group 7 | 8 | 124.2 ± 13.2 | 122.3 ± 8.4 | 127.8 ± 9.0 | 125.9 ± 10.0 |

### Test Example 3: Antihypertensive Effects

Male apolipoprotein E (ApoE) knock out mice aged 12 weeks were divided into 4 groups (15 mice per group) as following: control group (0.5% carboxymethylcellulose (CMC) solution administered group), olmesartan medoxomil (3 mg/kg) administered group, azelnidipine (3 mg/kg) administered group, and olmesartan medoxomil (3 mg/kg) plus azelnidipine (3 mg/kg) administered group. Either test substance or vehicle (0.5% CMC solution) was orally administered to animals for 24 successive weeks. A high fatty diet (containing 0.15% cholesterol and 15% unsalted butter) was given to all mice in all groups following the start of test agent administration (12 weeks of age). The systolic blood pressure of all mice was measured by a non-preheating type blood pressure monitor (BP MONITOR FOR RATS & MICE, Model MK-2000, Muromachi Kikai Co., Ltd.) at 21 - 24 hr after drug administration in the 23rd week. The results are shown in Table 3 (values in the table indicate mean ± S.E.).

**Table 3**

| Administration Group | Systolic blood pressures (mmHg) |
|---|---|
| Control group | 129 ± 3 |
| Olmesartan medoxomil administered group | 121 ± 4 |
| Azelnidipine administered group | 127 ± 4 |
| Olmesartan medoxomil and azelnidipine co-administered group | 112 ± 3 |

As the results show above, remarkable hypotensive effects (p = 0.0063; Dunnett's multiple comparison test) were observed in the olmesartan medoxomil and azelnidipine co-administered group, at which doses either agent alone did not elicit any significant effects, and the effects elicited by co-administration of olmesartan medoxomil and azelnidipine were synergistic (p = 0.0065; two-way analysis of variance).

### (Preparation example)

| Tablets (Combination Drug) | |
|---|---|
| Olmesartan medoxomil | 10.0 mg |
| Azelnidipine | 10.0 mg |
| Lactose | 278.0 mg |
| Corn Starch | 50.0 mg |
| Magnesium stearate | 2.0 mg |

The powders of above prescription are mixed and tableted with a tableting machine to prepare a tablet comprising 350 mg of the composition. The tablets can be sugar coated, when it is necessary.

### [Industrial applicability]

The medicament of the present invention is useful as a prophylactic and/or therapeutic agent against arteriosclerosis and hypertension.

## Claims

1. A medicament for the prevention and/or treatment of arteriosclerosis comprising the following composition;
(A) an angiotensin II receptor antagonist selected from the group consisting of a compound having a general formula (I), pharmacologically acceptable esters thereof and pharmacologically acceptable salts thereof; and 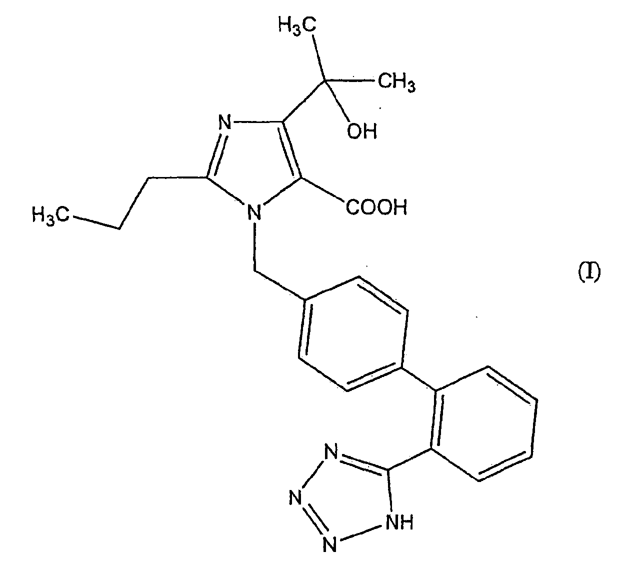
(B) a calcium channel blocker selected from the group consisting of 1,4-dihydropyridine derivatives and pharmacologically acceptable salts thereof as active ingredients.

2. A medicament for the inhibition of proliferation of vascular smooth muscle cells comprising the compound (A) and the compound (B) according to claim 1 as active ingredients.

3. A medicament for the inhibition of neointima formation of the blood vessels comprising the compound (A) and the compound (B) of claim 1 as active ingredients.

4. A medicament for the inhibition of vascular remodeling comprising the compound (A) and the compound (B) of claim 1 as active ingredients.

5. A medicament for the prevention of restenosis following percutaneous coronary intervention comprising the compound (A) and the compound (B) of claim 1 as active ingredients.

6. A medicament for the prophylaxis and/or treatment of hypertension or diseases caused by hypertension comprising the compound (A) and the compound (B) of claim 1 as active ingredients, wherein the medicament is suitable for administration of the compound (A) and the compound (B) at the same time or separately at certain intervals.

7. A medicament for the prophylaxis and/or treatment of hypertension comprising the compound (A) and the compound (B) of claim 1 as active ingredients.

8. A medicament for the prophylaxis and/or treatment of heart diseases comprising the compound (A) and the compound (B) of claim 1 as active ingredients.

9. A medicament for the prophylaxis and/or treatment of angina pectoris comprising the compound (A) and the compound (B) of claim 1 as active ingredients.

10. A medicament for the prophylaxis and/or treatment of myocardial infarction comprising the compound (A) and the compound (B) of claim 1 as active ingredients.

11. A medicament for the prophylaxis and/or treatment of arrhythmia comprising the compound (A) and the compound (B) of claim 1 as active ingredients.

12. A medicament for the prophylaxis of sudden death comprising the compound (A) and the compound (B) of claim 1 as active ingredients.

13. A medicament for the prophylaxis and/or treatment of heart failure comprising the compound (A) and the compound (B) of claim 1 as active ingredients.

14. A medicament for the prophylaxis and/or treatment of cardiac hypertrophy comprising the compound (A) and the compound (B) of claim 1 as active ingredients.

15. A medicament for the prophylaxis and/or treatment of renal diseases comprising the compound (A) and the compound (B) of claim 1 as active ingredients.

16. A medicament for the prophylaxis and/or treatment of diabetic nephropathy comprising the compound (A) and the compound (B) of claim 1 as active ingredients.

17. A medicament for the prophylaxis and/or treatment of glomerulonephritis comprising the compound (A) and the compound (B) of claim 1 as active ingredients.

18. A medicament for the prophylaxis and/or treatment of nephrosclerosis comprising the compound (A) and the compound (B) of claim 1 as active ingredients.

19. A medicament for the prophylaxis and/or treatment of cerebrovascular diseases comprising the compound (A) and the compound (B) of claim 1 as active ingredients.

20. A medicament for the prophylaxis and/or treatment of cerebral infarction comprising the compound (A) and the compound (B) of claim 1 as active ingredients.

21. A medicament for the prophylaxis and/or treatment of cerebral hemorrhage comprising the compound (A) and the compound (B) of claim 1 as active ingredients.

22. A medicament according to any one claims 1 to 21, wherein the medicament is a pharmaceutical composition comprising the compound (A) and the compound (B) as active ingredients.

23. A medicament according to any one claims 1 to 21, wherein the compound (A) and the compound (B) are administered at the same time or separately at certain intervals.

24. A medicament according to any one claims 1 to 21, wherein the angiotensin II receptor antagonist is (5-methyl-2-oxo-1,3-dioxolen-4-yl)methyl 4-(1-hydroxy-1-methylethyl)-2-propyl-1-[[2'-(1H-tetrazol-5-yl)biphenyl-4-yl]methyl]imidazol-5-carboxylate.

25. A medicament according to any one claims 1 to 21, wherein the calcium channel blocker is selected from the group consisting of azelnidipine, amlodipine, benidipine, nitrendipine, manidipine, nicardipine, nifedipine, nisoldipine, cilnidipine, lercanidipine, niguldipine, nimodipine, aranidipine, efonidipine, bamidipine, felodipine, and nilvadipine.

26. A medicament according to any one claims 1 to 21, wherein the calcium channel blocker is azelnidipine.

27. A medicament according to any one claims 1 to 21, wherein the angiotensin II receptor antagonist is (5-methyl-2-oxo-1,3-dioxolen-4-yl)methyl 4-(1-hydroxy-1-methylethyl)-2-propyl-1-[[2'-(1H-tetrazol-5-yl)biphenyl-4-yl]methyl]-imidazol-5-carboxylate and the calcium channel blocker is azelnidipine.
